# EUROPÄISCHE PATENTSCHRIFT

(11) **EP 2 307 096 B1**
(45) Veröffentlichungstag und Bekanntmachung des Hinweises auf die Patenterteilung: **17.04.2019**
(21) Anmeldenummer: 09777535.7
(22) Anmeldetag: 30.07.2009
(51) Int. Cl.: A61N 5/10

(54) **VORRICHTUNG UND VERFAHREN ZUR AUSWERTUNG EINER AKTIVITÄTSVERTEILUNG SOWIE BESTRAHLUNGSANLAGE**
APPARATUS AND METHOD FOR EVALUATING AN ACTIVITY DISTRIBUTION, AND IRRADIATION SYSTEM
DISPOSITIF ET PROCÉDÉ POUR ÉVALUER UNE RÉPARTITION D'ACTIVITÉ ET INSTALLATION D'IRRADIATION

(30) Priorität: 05.08.2008 DE 102008036478
(43) Veröffentlichungstag der Anmeldung: 13.04.2011
(73) Patentinhaber: GSI Helmholtzzentrum für Schwerionenforschung GmbH, 64291 Darmstadt (DE); Helmholtz-Zentrum Dresden - Rossendorf e.V., 01328 Dresden (DE); Heidelberg Ionenstrahl-Therapie (HIT) Betriebs-Gesellschaft Am Universitätsklinikum Heidelberg MBH, 69120 Heidelberg (DE)
(72) Erfinder: BERT, Christoph, 63741 Aschaffenburg (DE); RIETZEL, Eike, 64331 Weiterstadt (DE); ENGHARDT, Wolfgang, 01454 Ullersdorf (DE); PARODI, Katia, 69117 Heidelberg (DE)
(74) Vertreter: Mergel, Volker
(86) Internationale Anmeldenummer: PCT/EP2009/005512
(87) Internationale Veröffentlichungsnummer: WO 2010/015358

(56) Entgegenhaltungen:
- WO-A2-2007/079854
- DE-B3-102006 024 243
- US-A1- 2007 055 090
- QIAO F ET AL: "Compensating Respiratory Motion in PET Image Reconstruction Using 4D PET/CT" NUCLEAR SCIENCE SYMPOSIUM CONFERENCE RECORD, 2005 IEEE WYNDHAM EL CONQUISTADOR RESORT, PUERTO RICO OCTOBER 23 - 29, 2005, PISCATAWAY, NJ, USA,IEEE, Bd. 5, 23. Oktober 2005 (2005-10-23), Seiten 2595-2598, XP010896199 ISBN: 978-0-7803-9221-2 in der Anmeldung erwähnt
- BUHLER P ET AL: "An Accurate Method for Correction of Head Movement in PET" IEEE TRANSACTIONS ON MEDICAL IMAGING, IEEE SERVICE CENTER, PISCATAWAY, NJ, US, Bd. 23, Nr. 9, 1. September 2004 (2004-09-01), Seiten 1176-1185, XP011117922 ISSN: 0278-0062 in der Anmeldung erwähnt
- NEHMEH S A ET AL: "Respiratory Motion in Positron Emission Tomography/Computed Tomography: A Review" SEMINARS IN NUCLEAR MEDICINE, GRUNE AND STRATTON,, ORLANDO, FL, US, Bd. 38, Nr. 3, 1. Mai 2008 (2008-05-01), Seiten 167-176, XP022585220 ISSN: 0001-2998 [gefunden am 2008-04-04]
- BERT CHRISTOPH ET AL: "Target motion tracking with a scanned particle beam" MEDICAL PHYSICS, AIP, MELVILLE, NY, US, Bd. 34, Nr. 12, 20. November 2007 (2007-11-20), Seiten 4768-4771, XP012103258 ISSN: 0094-2405
- QIANG LI ET AL: "Online compensation for target motion with scanned particle beams: simulation environment; Online compensation for target motion with scanned particle beams" PHYSICS IN MEDICINE AND BIOLOGY, TAYLOR AND FRANCIS LTD. LONDON, GB, Bd. 49, Nr. 14, 21. Juli 2004 (2004-07-21) , Seiten 3029-3046, XP020023788 ISSN: 0031-9155

## Beschreibung

Die Erfindung betrifft eine Bestrahlungsanlage mit einer Vorrichtung und ein Verfahren zur Auswertung einer erzeugten Aktivitätsverteilung. Die Ausführungsformen der Erfindung finden insbesondere im Rahmen der Partikeltherapie Einsatz und können dort zur Überwachung bzw. zur Verifikation der Aktivitätsverteilung bzw. der deponierten Dosisverteilung verwendet werden.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, insbesondere auch das Verfahren nach der unten dargestellten Erfindung, finden jedoch auch in nichttherapeutischen Gebieten Anwendung. Hierzu gehören beispielsweise Forschungsarbeiten, die an nicht-lebenden Phantomen oder Körpern, aber auch an *in-vitro* und *in-vivo* Systemen durchgeführt werden, Bestrahlungen von Materialien, etc. Hierbei werden geladene Partikel wie z.B. Protonen oder Kohlenstoffionen oder Ionen anderer Elemente auf hohe Energien beschleunigt, zu einem Partikelstrahl geformt und über ein Hochenergiestrahltransportsystem zu einem oder mehreren Bestrahlungsräumen geführt. In einem dieser Bestrahlungsräume wird das zu bestrahlende Objekt mit dem Partikelstrahl bestrahlt.

Abhängig von der Energie des Partikelstrahls dringt der Partikelstrahl in das Zielobjekt ein und wechselwirkt mit dem Zielobjekt in einem relativ eng umschriebenen Bereich, der vorzugsweise in einem zuvor festgelegten Zielvolumen liegt. Dies hat den Vorteil, dass ein das Zielvolumen umgebendes Gebiet verglichen mit anderen Bestrahlungsarten gut geschont werden kann. Insbesondere mit einem gescannten Partikelstrahl - d.h. mit einem Partikelstrahl, der sukzessive auf unterschiedliche Teilbereiche des Zielobjekts gerichtet wird (und das Zielobjekt so "scannt") - kann eine genaue Dosisapplikation erreicht werden.

Bei der Bestrahlung von bewegten Zielvolumina mit einem gescannten Partikelstrahl werden üblicherweise Methoden benötigt, um die Bewegung des Zielvolumens zu kompensieren, da ohne eine Berücksichtigung der Bewegung eine gewünschte Dosisverteilung im Zielobjekt oftmals nicht erreicht werden kann.

Bekannt sind das als Gating bezeichnete Verfahren, das als Tracking bezeichnete Verfahren, das als Rescanning bezeichnete Verfahren und die fraktionierte Bestrahlung mit einem durch die Bewegung gegebenen zusätzlichen Anforderungen genügenden Bestrahlungsplan.

Beim Gating wird ein Zeitfenster bestimmt, das auf die Bewegung des Zielvolumens abgestimmt ist und während dem eine Applikation des Strahls erfolgt, beim Tracking wird der Strahl der Bewegung des Zielvolumens nachgeführt, und beim Rescanning wird ein Zielvolumen mehrfach hintereinander bestrahlt, wodurch sich Bewegungseffekte ausmitteln.

Zudem wird bei Bestrahlungen gewöhnlich ein Sicherheitssaum um das Zielvolumen gelegt, um kleine Abweichungen sowie Lageänderungen des Zielvolumens von einer angenommenen Position des Zielvolumens zu kompensieren. Bei der Bestrahlung eines Patienten beispielsweise können Positionsänderungen des Patienten sowie Lageänderungen des zu bestrahlenden Gebiets zwischen verschiedenen Bestrahlungssitzungen (interfraktionelle Lageänderungen), aber auch während einer Bestrahlungssitzung (intrafraktionelle Lageänderungen) berücksichtigt werden.

Bei der Bestrahlung von stationären Tumoren - d.h. von Tumoren, welche sich während einer Bestrahlungssitzung nicht bewegen, wie z.B. bei Tumoren in der Schädelbasis - ist die Verwendung der so genannten in-beam Positronen-Emissions-Tomographie bekannt. Im Rahmen der Erfindung steht die Abkürzung PET für Positronen-Emissions-Tomographie. Mit einem PET-System werden die Photonen detektiert, die von β⁺-radioaktiven Nukliden emittiert werden. Die β⁺-radioaktiven Nuklide werden ihrerseits durch die Wechselwirkung des Partikelstrahls mit dem bestrahlten Material bzw. Gewebe erzeugt. Anschließend kann hieraus die Verteilung der Aktivierung rekonstruiert werden, welche durch den Partikelstrahl hervorgerufen wird.

Eine derartige Therapieüberwachung ist für stationäre Tumore in der Veröffentlichung Parodi K. et al., "The feasibility of in-beam PET for accurate monitoring of proton therapy: results of a comprehensive experimental study" IEEE Nuclear Science Symposium Conference Record, Volume 4, 2004, 2160 - 2164, bzw. in der Veröffentlichung
Parodi K. et al., "Experimental study on the feasibility of in-beam PET for accurate monitoring of proton therapy" IEEE Transactions on Nuclear Science, Volume 52, 2005, 778 - 786, oder in der Veröffentlichung Enghardt W. et al., "Charged hadron tumor therapy monitoring by means of PET" Nucl. Instrum. Meth. A525, 2004, 284 - 288 offenbart.

Aus der nuklearmedizinischen Diagnostik ist die Verwendung von 4D-PET bekannt, beispielsweise aus der Druckschrift Feng Qiao et al., "Compensating respiratory motion in PET image reconstruction using 4D PET/CT" IEEEn Nuclear Science Symposium Conference Record, Volume 5, 2005, 2595 - 2598. Hierbei können PET Daten beispielsweise derart rekonstruiert werden, dass eine Bewegungsinformation in den Rekonstruktionsprozess einfließt.

Aus der Schrift Bühler P et al., "An accurate method for correction of head movement in PET", IEEE Trans. Med. Imag. 23, 2004, 1176 - 1185 ist ein Verfahren zur Bewegungskorrektur von PET-Daten bekannt, in dem eine Bewegung des Kopfes während der Datenaufzeichnung berücksichtigt wird.

Aus der Veröffentlichung "Online compensation for target motion with scanned particle beams: simulation environment" von Qiang Li Et. Al., Phys. Med. Biol. 49 (2004), S. 3029 bis 3046, ist bekannt, dass die Zielbewegung eine der deutlichsten Beschränkungen für jede hochgenaue Strahlungstherapie darstellt. Die Wechselwirkung zwischen zeitabhängiger Strahl- und Zielposition kann demnach die applizierte Dosisverteilung stark beeinflussen.

Aus der Patentschrift DE 10 2006 024 243 B3 ist ferner bekannt, eine Bestrahlungsdosis mittels PET bei unbewegten Zielvolumen ortsaufgelöst zu kontrollieren.

Es ist die Aufgabe der Erfindung, eine Bestrahlungsanlage mit einer vorteilhaften Vorrichtung und ein vorteilhaftes Verfahren zur Auswertung einer in einem Zielobjekt durch einen Strahl erzeugten Aktivitätsverteilung bereitzustellen.

Die Aufgabe der Erfindung wird gelöst durch eine Bestrahlungsanlage gemäß Anspruch 1 sowie durch ein Verfahren gemäß Anspruch 8. Vorteilhafte Weiterbildungen der Erfindung finden sich in den Merkmalen der abhängigen Ansprüche.

Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist.

Die Bestrahlungsanlage gemäß Anspruch 1 weist eine Steuerungseinheit auf, mit welcher ein Bestrahlungsverlauf steuerbar ist, und ferner die erfindungsgemäße Vorrichtung zur Auswertung einer in einem bewegten Zielobjekt auftretenden Aktivitätsverteilung, welche durch den mit der Bestrahlungseinrichtung erzeugten Strahl erzeugbar ist. Die Vorrichtung weist folgende Merkmale auf:
- einen Positronen-Emissions-Tomograph, der insbesondere gegenüberliegend angeordnete Detektoren aufweist, und welcher ausgebildet ist zum Aufzeichnen von durch den Strahl im Zielobjekt erzeugten Photonen und zur Erzeugung von Messdaten, welche Entstehungsorte der Photonen repräsentieren,
- eine Bewegungserfassungseinrichtung, welche ausgebildet ist, ein Bewegungssignal zu erzeugen, das die Bewegung des Zielobjekts repräsentiert,
- Eine Datenaufnahmeeinheit zur Steuerung der Datenaufnahme des Positronen-Emissions-Tomographen mit einem ersten Eingang zur Erfassung der Messdaten des Positronen-Emissions-Tomographen, einem zweiten Eingang zum Eingeben von die Strahleigenschaften betreffenden Daten in die Datenaufnahmeeinheit, einem dritten Eingang, der mit der zweiten Steuerungseinheit der Bestrahlungseinrichtung verbunden ist, zum Eingeben von Strahlparametern, welche zur Steuerung des Partikelstrahls verwendet werden, in die Datenaufnahmeeinheit, einem vierten Eingang zur Erfassung des mit der Bewegungserfassungseinrichtung erzeugten Bewegungssignals, wobei die Datenaufnahmeeinheit ferner hergerichtet ist zur zeitlich korrelierten Speicherung der an den Eingängen erfassten Signale und Messdaten,
- eine Auswertungseinheit, welche ausgebildet ist, die Entstehungsorte der gemessenen Photonen zu Positionen im Zielobjekt unter Verwendung des Bewegungssignals zuzuordnen, wodurch eine räumliche Charakteristik der tatsächlich im Zielobjekt erzeugten Aktivitätsverteilung unter Verwendung der durch den Strahl erzeugten Photonen zeitaufgelöst und bewegungskorrigiert auswertbar ist.

Die Steuerung des Bestrahlungsverlaufs durch die zweite Steuerungseinheit bewirkt dabei gleichzeitig die Steuerung der Datenaufzeichnung mit dem Positronen-Emissions-Tomographen durch die Datenaufnahmeeinheit bewirkt, so dass die mit der Auswertungseinheit ermittelte zeitaufgelöste und bewegungskorrigierte Aktivitätsverteilung zu dem Bestrahlungsverlauf in Beziehung gesetzt werden kann
Mit dieser Vorrichtung kann im Gegensatz zu Vorrichtungen im Stand der Technik eine Überwachung der durch den Strahl im Zielobjekt erzeugten ß⁺-Emitter, welche in einem zweiten Schritt Photonen erzeugen, erfolgen, selbst wenn sich das zu bestrahlende Zielobjekt bewegt. Durch den Strahl wird im Zielobjekt folglich eine Aktivitätsverteilung induziert, welche ihrerseits Photonen erzeugt, die durch den Positronen-Emissions-Tomographen gemessen werden können. Eine tatsächlich im Zielobjekt deponierte Dosisverteilung kann über die erzeugte Aktivitätsverteilung, und somit über die durch den Strahl im Zielobjekt erzeugten Photonen, verfolgt werden.

Bei Vorrichtungen im Stand der Technik ist eine Überwachung in dem Fall des bewegten Zielobjekts nicht möglich oder zumindest unzureichend, da die Bewegung des Zielobjekts eine Unscharfe erzeugt, wodurch es unmöglich ist, die tatsächlich erzeugte Aktivitätsverteilung und damit die tatsächlich deponierte Dosisverteilung korrekt auszuwerten. Bei einem bewegten Tumor z.B. betrifft die bewegte Anatomie auch die räumliche Lage und gegebenenfalls die räumliche Verteilung der ß⁺-Emitter. Eine Messung der durch den Strahl induzierten Aktivitätsverteilung wird demnach aufgrund der Bewegung verfälscht. Dies führt dann zu einer ungenauen, für gewöhnlich verwaschenen Rekonstruktion der Aktivitätsverteilung. Wenn aus der Aktivitätsverteilung dann auf die tatsächlich deponierte Dosisverteilung geschlossen werden soll, würde diese Unscharfe der Aktivitätsverteilung verhindern, eine räumliche Charakteristik der deponierten Dosisverteilung zu erzeugen.

Falls z.B. eine Abweichung zwischen der gemessenen Aktivitätsverteilung und einer Sollaktivitätsverteilung bzw. zwischen der hieraus ermittelten Dosisverteilung und einer Solldosisverteilung festgestellt wird, kann im herkömmlichen Falle gemäß dem Stand der Technik nicht zugeordnet werden, ob diese Abweichung auf eine fehlerhafte Deposition der Dosisverteilung zurückzuführen ist, oder ob eine Bewegung des Zielobjekts bei der Aufzeichnung der emittierten Photonen die Ursache für die Abweichung ist.

Bei der erfindungsgemäßen Vorrichtung werden durch die Auswertungseinheit die Positionen im Zielobjekt bestimmt, an denen die mittels der PET-Detektoren gemessenen Photonen entstanden sind, und zwar unter Verwendung des Bewegungssignals. Hierdurch kann die Bewegung des Zielobjekts bei der Auswertung der Messdaten berücksichtigt werden. Bei der Auswertung werden folglich aus den Messdaten bewegungskorrigierte Daten ermittelt. Dies bedeutet, dass bei einer Rekonstruktion der Aktivitätsverteilung die Bewegung des Zielobjekts berücksichtigt wird. Hierdurch wird eine verbesserte Rekonstruktion möglich, vorzugsweise kann eine weitgehend perfekte Rekonstruktion erreicht werden.

Diese räumliche Bestimmung der Entstehungsorte der Photonen bezogen auf das Zielobjekt erlaubt es somit, die räumliche Charakteristik der deponierten Dosisverteilung - d.h. unter Verwendung der durch den Strahl erzeugten Aktivitätsverteilung - auch in einem räumlich bewegten Zielobjekt zu bestimmen. Wenn nun eine Abweichung der derart bestimmten Aktivitätsverteilung von einer Sollaktivitätsverteilung auftritt, kann daraus geschlossen werden, dass diese Abweichung durch eine fehlerhafte Deposition der Dosis im Zielobjekt bedingt ist. Die gemessene und bewegungskorrigierte Aktivitätsverteilung erlaubt folglich, die tatsächlich deponierte Dosisverteilung besser zu beurteilen. Dabei kann vornehmlich eine räumliche Charakteristik der gemessenen Aktivitätsverteilung ermittelt werden.

Bei der Bestimmung der Positionen im Zielobjekt, an denen die gemessenen Photonen entstanden sind, wird das Bewegungssignal verwendet. Mithilfe des Bewegungssignals wird eine Korrelation zwischen der Bewegung des Zielobjekts, also der räumlichen Lage des Zielobjekts als Funktion der Zeit, und dem PET-System bestimmt, wodurch die räumliche Zuordnung der Entstehungsorte der durch den Strahl induzierten Photonen zu Positionen im Zielobjekt erst ermöglicht wird. Die Messdaten und das Bewegungssignal werden derart z.B. aufgezeichnet, dass hieraus eine zeitliche Korrelation zwischen den Messdaten und dem Bewegungssignal herstellbar ist. Unter Korrelation wird hierbei verstanden, dass die zwei zeitlich veränderlichen Größen zueinander in Beziehung gesetzt werden; eine Beschränkung auf das zweite Moment der gemeinsamen Verteilung ist durch das Wort "Korrelation" hier nicht beabsichtigt, insofern wird das Wort "Korrelation" in der Anmeldung allgemeiner als in der Statistik definiert verwendet; dies dient der sprachlichen Einfachheit.

Gewöhnlich werden derartige ß⁺-Emitter vornehmlich in ausreichender Anzahl durch einen Partikelstrahl wie beispielsweise Protonen, Kohlenstoffionen oder auch Ionen anderer Elemente erzeugt. Denkbar sind auch Ionen von Elementen, welche radioaktiv sind und die selbst einen ß⁺-Emitter darstellen. Es ist jedoch sogar denkbar, dass derartige ß⁺-Emitter durch harte Röntgenstrahlung erzeugt werden. Vergleiche hierzu: Kluge T. et al., "First in-beam PET measurement of ß+-radioactivity induced by hard photon beams", Phys. Med. Biol. 52, 2007, N467-N473.

Die Bewegungserfassungseinrichtung kann unterschiedlich ausgebildet sein und gegebenenfalls der Komplexität der Bewegung des Zielobjekts und der erforderlichen Genauigkeit der Detektion angepasst sein.

Die Bewegungserfassungseinrichtung kann in einem einfachen Fall ein Sensor sein, mit welchem eine Größe gemessen wird, die kennzeichnend für die Bewegung ist, also eine Ersatzgröße (Surrogatgröße). Ein derartiger Sensor kann beispielsweise den Atemfluss (z.B. über die Atemtemperatur), die Bewegung des Brustkorbs (z.B. über den Umfang des Brustkorbes) messen, usw., woraus ein Signal erzeugt wird, aus welchem die Bewegung des Zielobjekts ermittelt werden kann. Die gemessenen PET-Daten können so einzelnen Bewegungsphasen zugeordnet werden. Dies kann z.B. retrospektiv erfolgen. Es kann aber auch möglich sein, mit dem Sensor die Bewegung vor und/oder während der Erzeugung der Photonen zu detektieren.

Die Bewegungserfassungseinrichtung kann in einer komplexeren Ausgestaltung auch eine Bildgebungseinrichtung, z.B. ein Fluoroskopiesystem sein, mit welchem die Bewegung des zu bestrahlenden Zielobjekts und insbesondere des Zielvolumens innerhalb des Zielobjekts direkt beobachtet werden kann. Weitere Ausgestaltungen werden ferner in der nachfolgenden Beschreibung beschrieben.

Die Bewegungserfassungseinrichtung kann jedoch auch lediglich eine dem PET-System zugeordnete Einheit sein, welche ein Signal erzeugt, das die Bewegung des Zielobjekts kennzeichnet. Input für diese Einheit können beispielsweise das Sensorsignal oder die von der Bildgebungseinrichtung erzeugten Daten sein. Das durch die Einheit erzeugte Bewegungssignal repräsentiert dann die außerhalb der Einheit erfassten Bewegungen des Zielvolumens.

### Beispielsweise für den

Fall, dass die Bewegung des Zielobjekts sehr regelmäßig ist, kann es aber auch ausreichen, dass diese Einheit ein Signal erzeugt, welches lediglich eine zu erwartende Bewegung des Zielobjekts repräsentiert. In diesem Fall wäre eine kontinuierliche Überwachung der Bewegung des Zielobjekts über eine externe Vorrichtung wie einen Sensor, ein Bildgebungssystem etc. nicht notwendig.

Insbesondere kann die Bewegungserfassungseinrichtung ein Bewegungssignal liefern, welches die Bewegung des Zielobjekts während der Bestrahlung repräsentiert. Die Erfassung der Messdaten mit dem Positronen-Emissions-Tomographen kann dann ebenfalls während der Bestrahlung erfolgen.

Erfindungsgemäß weist der Positronen-Emissions-Tomograph eine erste Steuerungseinheit auf, welche mit einer zweiten Steuerungseinheit der Bestrahlungseinrichtung in Wirkverbindung steht, wobei mit der zweiten Steuerungseinheit ein Verlauf der Bestrahlung steuerbar ist. Mit der ersten Steuerungseinheit kann die Datenaufnahme des Positronen-Emissions-Tomographen gesteuert werden. Dadurch, dass die erste Steuerungseinheit in Wirkverbindung mit einer Steuerungseinheit steht, welche den Verlauf der Bestrahlung steuert, beispielsweise den Scan-Verlauf, ist es nun möglich, die Datenaufzeichnung mit dem Bestrahlungsverlauf zeitlich abzustimmen. Eine Steuerung des Bestrahlungsverlaufs, z.B. des Scan-Verlaufs, bewirkt gleichzeitig eine Steuerung der Datenaufzeichnung mit dem Positronen-Emissions-Tomographen.

Denkbar sind Ausgestaltungen, bei denen der Positronen-Emissions-Tomograph erst nach abgeschlossener Bestrahlung des Zielvolumens eingesetzt wird, um die Daten aufzuzeichnen. Dies ist deshalb möglich, da die durch einen Strahl erzeugte Aktivierung nicht sofort abklingt, sondern für eine gewisse Zeit noch detektierbar ist. Die Datenaufnahme mit dem PET-System könnte nach Abschluss der Bestrahlung auch manuell gestartet werden. Das PET-System kann dabei sowohl im Bestrahlungsraum angeordnet sein als auch in einem separaten Raum. Es können auch mehrere radioaktive Nuklide mit unterschiedlich langen Zerfallszeiten durch die Bestrahlung mit dem Partikelstrahl erzeugt werden. Weiterhin ist es auch möglich, sowohl während der Bestrahlung, als auch nach Abschluss der Bestrahlung eine Aktivitätsverteilung mit dem PET-System zu bestimmen.

Die Aufzeichnung der Messdaten mit dem PET-System während der Bestrahlung weist jedoch gegenüber einer nachträglichen Aufzeichnung den Vorteil auf, dass die gemessene Aktivitätsverteilung weniger sensitiv auf physiologische Vorgänge - z.B. einen Washout durch den Blutfluss - ist, die wiederum zu einer Veränderung der deponierten Aktivitätsverteilung führen. Die Aktivitätsverteilung verändert sich folglich als Funktion der Zeit nach Abschluss der Bestrahlung.

In einer vorteilhaften Ausführungsform ist die Auswertungseinheit zusätzlich dazu ausgebildet, die gemessenen Photonen zeitlich mit einem Verlauf der Bestrahlung zu korrelieren. Beispielsweise kann eine zeitliche Korrelation über die Signale des Bestrahlungsvorgangs (Zeitpunkt von Bestrahlungsabschnitten wie beispielsweise der Übergang zu einem neuen Bestrahlungspunkt), mit den Messdaten des PET-Systems bestimmt werden. Hierdurch kann auch ein zeitlicher Bezug zur Applikation des Bestrahlungsplan gewährleistet sein. Auf diese Weise kann die zeitliche Entstehung der Aktivitätsverteilung, welche im Zielobjekt durch die Bestrahlung sukzessive entsteht, ausgewertet werden, und insbesondere mit dem Bestrahlungsverlauf verglichen werden. Folglich ist eine Auswertung des dynamischen 4D-Bestrahlungsablaufs möglich.

Hierbei kann die im bewegten Zielobjekt erzeugte Aktivitätsverteilung bestimmt und mit dem Bestrahlungsverlauf in Beziehung gesetzt werden, insbesondere mit den zeitlich und/oder örtlich sukzessive deponierten Einzeldosen. Auf diese Weise kann z.B. die räumliche Charakteristik der deponierten Dosisverteilung im Zielobjekt zu verschiedenen Zeitpunkten während der Bestrahlung ermittelt und ausgewertet werden und jeweils zu zugehörigen Bestrahlungsphasen in Beziehung gesetzt werden.

Es ist z.B. denkbar, dass bei einem Scan-Verfahren beispielsweise die inkrementell anwachsende Aktivitätsverteilung - oder die sukzessive deponierte Dosisverteilung - im Zielobjekt gemessen werden kann und mit dem Scan-Verlauf in Beziehung gesetzt wird. Hierdurch ist es zumindest teilweise möglich zu bestimmen, welche Dosis wo im Zielobjekt deponiert wurde, selbst wenn sich das Zielobjekt bewegt. Es kann also eine Korrelation der PET-Messdaten mit zumindest einem Teil der Rasterpunkte, mit zumindest einem Teil der Iso-Energieschicht oder mit zumindest einem Teil der Spills - oder auch Gruppen oder Kombinationen hiervon - zur Überwachung bzw. zur Rekonstruktion des dynamischen, 4D-Bestrahlungsablaufs vorgenommen werden. Dies gilt insbesondere dann, wenn eine Dosis in einem Gebiet des Zielobjekts deponiert wird, welches zuvor nicht bestrahlt worden ist, beispielsweise hinter einer distalen Kante eines bereits bestrahlten Gebiets, beim Übergang von einer Iso-Energieschicht zu einer anderen Iso-Energieschicht oder wenn eine Dosis in Strahlrichtung hinter dem bereits teilweise bestrahlten Zielvolumen fälschlicherweise deponiert wird. Hierdurch können sich z.B. "Ausreißer" detektieren und zuordnen lassen.

Üblicherweise wird dies dadurch erreicht, dass das Bewegungssignal, die Datenerfassung des Positronen-Emissions-Tomographen und ggf. der Fortschritt der Bestrahlung derart aufgezeichnet werden, dass die zugeordneten Datenmengen zeitlich miteinander korrelierbar sind, so dass die Auswertungseinheit die notwendige zeitliche Korrelation auf einfache Weise herstellen kann. Dies kann z.B. dadurch erreicht werden, dass im Verlauf der Datenaufzeichnung die Daten zeitlich korreliert in einer Datensammlungseinheit abgespeichert werden.

In einer anderen Ausführungsvariante ist die Auswertungseinheit ausgebildet, um das Bewegungssignal aus den aufgezeichneten Messdaten zu ermitteln, insbesondere mit Hilfe eines radioaktiven Markers, der durch die Bewegung des Zielobjekts bewegt wird. In diesem Fall fungiert das PET-System selbst als Bewegungserfassungseinrichtung.

Alternativ oder zusätzlich ist es möglich, dass die Auswertungseinheit ausgebildet ist, um ein weiteres Bewegungssignal, welches ebenso die Bewegung des Zielobjekts kennzeichnet, aus den Messdaten zu ermitteln, und somit zusätzlich zu dem Bewegungssignal, das von einer Bewegungserfassungseinrichtung bereitgestellt wird.

Die Bewegung des Zielobjekts kann z.B. durch einen radioaktiven Marker, der durch das Zielobjekt bewegt wird und dessen Bewegung durch den Positronen-Emissions-Tomographen erfasst wird, detektiert werden. Ein oder mehrere Marker können insbesondere mit dem Zielobjekt, z.B. auf dessen Oberfläche, fest verbunden sein und können eine möglichst punktförmige Strahlungsquelle aufweisen.

In einer vorteilhaften Ausführungsform ist die Auswertungseinheit zusätzlich derart ausgebildet, aus einer Intensität der gemessenen Photonen quantitativ ein Maß für die deponierte Dosisverteilung an den Positionen im Zielobjekt zu ermitteln. Dies ist nun möglich, da bei einem bewegten Zielobjekt die erzeugte Aktivitätsverteilung zeitlich und örtlich genau zu Positionen im Zielobjekt zugeordnet werden kann. Es ist daher denkbar, die mithilfe des Positronen-Emissions-Tomographen gemessenen Photonen dazu zu verwenden, den Grad der Aktivierung an einem bestimmten Ort im Zielobjekt zu bestimmen, und hierüber die deponierte Dosisverteilung auch quantitativ - zumindest annähernd quantitativ - und nicht nur lediglich bezüglich ihrer räumlichen Ausdehnung zu rekonstruieren.

Darüber hinaus kann in einer vorteilhaften Weise die Vorrichtung zusätzlich eine Beurteilungseinheit aufweisen, welche derart ausgebildet ist, dass die ermittelte Aktivitätsverteilung mit einer charakteristischen Größe einer Bestrahlungsplanung verglichen wird.

Dies kann beispielsweise die räumliche Ausdehnung der Aktivitätsverteilung oder der hieraus ermittelbaren, tatsächlich deponierten Dosisverteilung bezogen auf den Behandlungs- bzw. Therapieplan sein. Dies kann aber auch ein aus der Bestrahlungsplanung generierte Vorhersagewert sein, welcher die zu erwartende Dosisverteilung oder die zu erwartende Aktivitätsverteilung wiedergibt, die mithilfe des Positronen-Emissions-Tomographen ermittelbar bzw. messbar sein sollte. Diese zu erwartende Dosisverteilung/Aktivitätsverteilung kann dann mit der tatsächlich gemessenen Dosisverteilung/Aktivitätsverteilung so verglichen werden, dass auf einfache Art und Weise Abweichungen detektiert werden können.

Um einem Anwender den Vergleich zu erleichtern, kann eine Darstellungseinheit vorgesehen sein, mit welcher die charakteristische Größe der Bestrahlungsplanung und die räumliche Charakteristik der Aktivitätsverteilung gemeinsam einem Anwender dargestellt werden. Beispielsweise kann eine Abbildung aus der Bestrahlungsplanung und die räumliche Charakteristik der Dosisverteilung zueinander skaliert dargestellt werden. Dabei können die beiden Abbildungen überlagert oder beispielsweise auch nebeneinander dargestellt werden, so dass die Abbildungen unmittelbar miteinander verglichen werden können. Auf diese Weise kann beispielsweise eine Verifikation von Sicherheitssäumen einfach und effektiv durchgeführt werden.

In vorteilhafter Weise kann die Auswertungseinheit dabei zusätzlich so ausgebildet sein, dass - basierend auf den Messdaten, welche durch den Positronen-Emissions-Tomographen detektiert worden sind - ein Bestrahlungsverlauf in der vorzugsweise als Partikelstrahlanlage ausgebildete Bestrahlungsanlage gesteuert werden kann.

Bei dem erfindungsgemäßen Verfahren zur Auswertung einer im nicht belebten Zielobjekt erzeugten Aktivitätsverteilung wird unter Verwendung einer beliebig ausgestalteten Vorrichtung gemäß der Erfindung eine räumliche Charakteristik der im Zielobjekt durch einen Strahl erzeugten Aktivitätsverteilung mit Hilfe einer räumlichen Verteilung von Photonen, welche zeitlich aufgelöst gemessen werden, bestimmt. In vorteilhafter Weise kann zusätzlich aus einer Intensität der gemessenen Photonen quantitativ ein Maß für die tatsächlich deponierte Dosisverteilung an den Positionen im Zielobjekt ermittelt werden. In einer vorteilhaften Ausgestaltung kann aus der Verteilung der zeitlich aufgelöst gemessenen Photonen ebenfalls und/oder zusätzlich sogar eine Bewegung des Zielobjekts ermittelt werden. Die zeitlich aufgelöst gemessenen Photonen können sogar dazu verwendet werden, eine Bestrahlung bzw. den Bestrahlungsverlauf zu steuern.

Wenn die Photonen zeitlich aufgelöst gemessen werden, können die Photonen verschiedenen Bewegungsphasen zugeordnet werden. Insbesondere werden die Photonen dadurch mit diesen korreliert. Die räumliche Charakteristik der im Zielobjekt erzeugten Aktivitätsverteilung kann dann für verschiedene Bewegungsphasen separat ausgewertet werden.

Bei der Auswertung kann aus einem Bestrahlungsplan eine zu erwartende Verteilung der Photonen bestimmt werden. Für den statischen Fall ist dies in der Druckschrift Pönisch F et al., "The modelling of positron emitter production and PET imaging during carbon ion therapy", Phys. Med. Biol. 49, 2004, 5217-5232 offenbart. Bei Systemen gemäß dem Stand der Technik versagt jedoch die Anwendung auf ein bewegtes Zielobjekt, da die entstehende Unscharfe einen Vergleich verhindert. Erfindungsgemäß kann die zu erwartende Verteilung dann mit der tatsächlich gemessenen räumlichen Verteilung von Photonen verglichen werden, so dass eine Abweichung der Bestrahlung von einem Sollzustand einfach detektiert werden kann.

Anders formuliert betrifft die vorliegende Beschreibung grundsätzlich auch eine Vorrichtung zur Schätzung einer mit einem Strahl erzeugten Aktivitätsverteilung in einem bewegten Zielobjekt mit: einem Positronen-Emissions-Tomographen zum Messen von durch den Strahl im Zielobjekt erzeugten Photonen, einer Bewegungserfassungseinrichtung zum Erzeugen eines die Bewegung des Zielobjekts repräsentierenden Bewegungssignals und einer Auswertungseinheit zum gemeinsamen Auswerten von Messungen des Positronen-Emissions-Tomographen und des Bewegungssignals zur Schätzung einer bewegungskorrigierten Aktivitätsverteilung.

Grundsätzlich betrifft die Erfindung ebenso auch ein Verfahren zur Schätzung einer Aktivitätsverteilung in einem bewegten und nicht belebten Zielobjekt, mit den Schritten: Erzeugen der Aktivitätsverteilung in dem bewegten Zielobjekt mit einem Strahl, Messen von durch den Strahl in dem Zielobjekt erzeugten Photonen mit einem Positronen-Emissions-Tomographen, Erzeugen eines die Bewegung des Zielobjekts repräsentierenden Bewegungssignals mit einer Bewegungserfassungseinrichtung und gemeinsames Auswerten von Messungen des Positronen-Emissions-Tomographen und des Bewegungssignals zur Schätzung einer bewegungskorrigierten Aktivitätsverteilung.

Ausführungsformen der Erfindung und Weiterbildungen gemäß den Merkmalen der abhängigen Ansprüche werden anhand der nachfolgenden Zeichnung näher erläutert, ohne jedoch darauf beschränkt zu sein. Es zeigen:
Fig. 1 einen schematischen Aufbau einer Partikelstrahlanlage,
Fig. 2 einen schematischen Aufbau eines PET-Systems,
Fig. 3 eine Abtastung eines Zielvolumens mit einem Scan-Verfahren,
Fig. 4 ein Ablaufdiagramm einer Ausführungsform des Verfahrens.

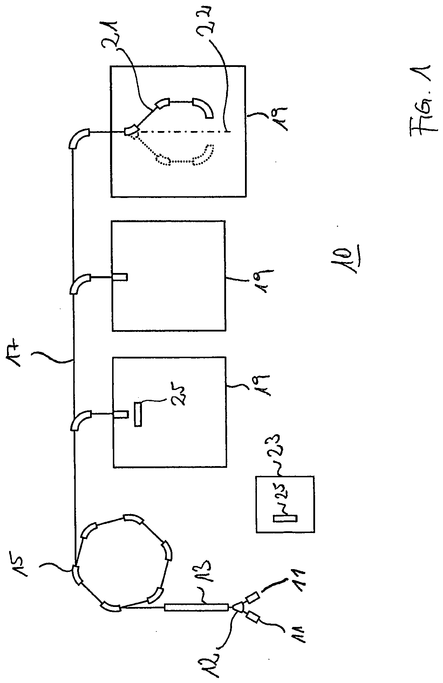

In den Figuren 1 bis 4 sind gleiche Teile stets mit den gleichen Bezugszeichen bezeichnet.

Figur 1 zeigt eine schematische Darstellung über einen beispielhaften Aufbau einer Partikelstrahlanlage 10. In einer Partikelstrahlanlage 10 erfolgt eine Bestrahlung eines Zielobjekts, insbesondere eine Bestrahlung eines menschlichen oder tierischen Körpers, insbesondere eines tumorerkrankten Gewebes, mit einem Partikelstrahl. Es kann aber auch ein Material oder ein als Phantom bezeichnetes Objekt bestrahlt werden.

Als Partikel werden vornehmlich Ionen wie beispielsweise Protonen, Heliumionen, Kohlenstoffionen, Ionen anderer Elemente oder andere Partikel wie Pionen eingesetzt. Üblicherweise werden derartige Partikel in einer Partikelquelle 11 erzeugt. Die Partikelquelle 11 kann z.B. eine Ionenquelle sein. Wenn, wie in Figur 1 dargestellt, zwei Partikelquellen 11 vorhanden sind, die zwei verschiedene Ionen verschiedener Elemente erzeugen, kann zwischen diesen beiden Arten von Ionen innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikelstrahlanlage 10 mit Protonen und mit Kohlenstoffionen im Wesentlichen gleichzeitig betrieben werden, insbesondere in kurz aufeinanderfolgenden Zeitintervallen abwechselnd betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise einen Kreisbeschleuniger wie ein Synchrotron oder Zyklotron, eingespeist. In dem Beschleuniger 15 werden die Ionen auf höhere Energien, wie sie zur Bestrahlung nötig sind, beschleunigt. Nachdem die Partikel den Beschleuniger 15 verlassen haben, führt ein Hochenergiestrahl-Transportsystem 17 den Partikelstrahl zu einem oder mehreren Bestrahlungsräumen 19. In dem Bestrahlungsraum 19 werden die beschleunigten Partikel auf ein zu bestrahlendes, hier nicht dargestelltes Zielobjekt gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen bezogen auf die Achse 22 aus.

Im Bestrahlungsraum 19 tritt der Partikelstrahl aus dem Strahltransportsystem aus 17 und trifft auf ein zu bestrahlendes Zielobjekt, das sich üblicherweise im Isozentrum eines Bestrahlungsraums befindet. In dem Zielobjekt befindet sich das zu bestrahlende Zielvolumen.

Der bisher beschriebene Grundaufbau einer Partikelstrahlanlage 10 ist beispielhaft für Partikelstrahlanlagen, kann aber auch hiervon abweichen. Somit ist der Aufbau der Partikelstrahlanlage nicht beschränkend für den Umfang der Vorrichtung, des Verfahrens sowie der Bestrahlungsanlage zu verstehen. Änderungen, Ergänzungen und/oder Äquivalente liegen im Umfang der Erfindung.

In einem oder mehreren Bestrahlungsräumen 19 oder aber auch in einem anderen Raum 23 ist ein PET-System 25 installiert. Die Funktionsweise des PET-Systems 25 wird anhand der nachfolgenden Figuren näher erläutert.

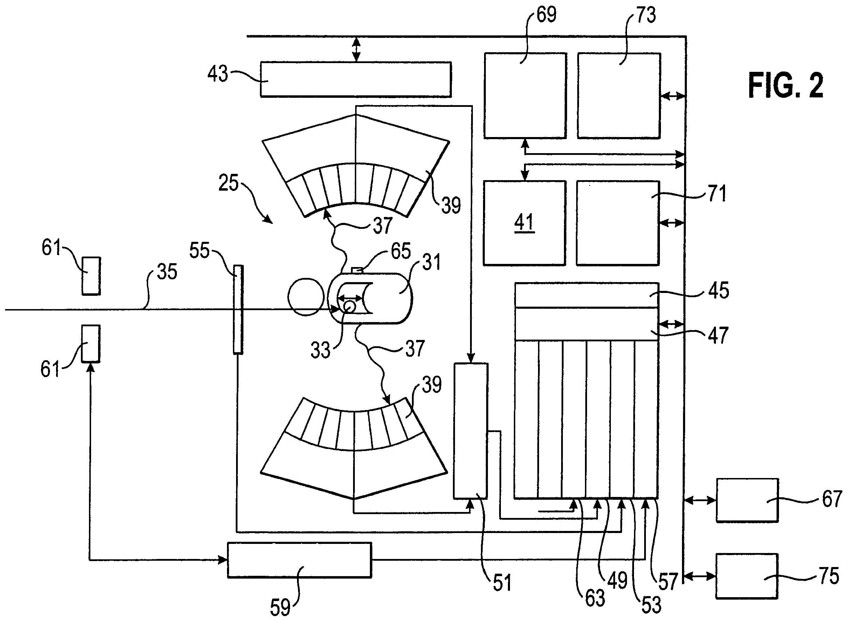

Fig. 2 zeigt den schematischen Aufbau eines PET-Systems 25, das in einem der Bestrahlungsräume 19 eingesetzt werden kann und das mit Komponenten der Partikelstrahlanlage 10 in Wirkverbindung und/oder Wechselwirkung steht.

Das PET-System 25 dient zur Auswertung einer in einem Zielobjekt 31, beispielsweise in einem Patienten oder in einem Phantom, erzeugten Aktivitätsverteilung, welche Rückschlüsse auf die tatsächlich im Zielobjekt 31 deponierte Dosisverteilung zulässt. Das Zielvolumen 33, beispielsweise ein Tumor oder ein festgelegter, umschriebener Bereich in einem Phantom, bewegt sich während der Bestrahlung und während der Aufzeichnung der Messdaten mit dem PET-System 25. Ist das Zielobjekt 31 ein Patient, kann die Bewegung beispielsweise durch eine Atembewegung bedingt sein.

In dem Zielvolumen 33 wird eine zuvor definierte Dosisverteilung mithilfe eines Partikelstrahls 35 deponiert. Der im Zielvolumen 33 applizierte Partikelstrahl wechselwirkt mit dem Material des Zielvolumens 33 und erzeugt eine Verteilung von β⁺-Emittern, die ihrerseits Photonen 37 emittieren, die in entgegengesetzter Richtung aus dem Zielvolumen 33 austreten. Die entgegengesetzt austretenden Photonen 37 werden mithilfe von zwei Detektorblöcken 39, die im Wesentlichen gegenüber liegend um das Zielvolumen 33 angeordnet sind, detektiert.

Das PET-System 25 wird von einer Steuerungseinheit 41 gesteuert. Diese Steuerungseinheit 41 ist mit anderen Untereinheiten des PET-Systems 25 gekoppelt.

Die hier dargestellte Aufteilung der einzelnen Einheiten und Untereinheiten des PET-Systems 25 und der dargestellten Einheiten der Beschleunigeranlage 10 ist lediglich eine Möglichkeit. Auch andere Aufteilungen sind möglich, beispielsweise können die verschiedenen Funktionalitäten in einer einzigen Steuereinheit integriert sein oder in anderer Weise auf Untereinheiten, welche miteinander gekoppelt sind, aufgeteilt werden.

Zu diesen Untereinheiten gehört beispielsweise eine Bewegungssteuerungseinheit 43, mit der die Rotation und Translation der PET-Gantry gesteuert werden kann. Auf diese Weise kann das PET-System 25 passend positioniert werden.

Zu diesen Untereinheiten gehört auch eine Datenaufnahmeeinheit 45, welche die Datenaufnahme des PET-Systems 25 steuert. Die Datenaufnahmeeinheit 45 verfügt über eine Datensammlungseinheit 47, welche unter anderem zur Speicherung der aufgezeichneten Messdaten dient.

Die Datenaufnahmeeinheit 45 weist hierfür einen ersten Eingang 49 auf, über welchen Messdaten in die Datenaufnahmeeinheit 45 weitergeleitet werden, die von den Detektorblöcken 39 aufgezeichnet und die mithilfe eines Koinzidenz-Prozessors 51 bezüglich einer zeitlichen Koinzidenz der detektierten Photonen ausgewertet worden sind. Die Aufzeichnung der Messdaten erfolgt hierbei insbesondere zeitaufgelöst.

Weiterhin verfügt die Datenaufnahmeeinheit 45 über einen zweiten Eingang 53, der mit einem Strahldiagnosesystem 55 in Verbindung steht bzw. gekoppelt ist. Über diesen zweiten Eingang 53 können Daten, die Strahleigenschaften betreffen, in die Datenaufnahmeeinheit 45 eingegeben und abgespeichert werden.

Weiterhin verfügt die Datenaufnahmeeinheit 45 über einen dritten Eingang 57, welcher mit einer Steuerungseinheit 59 der Partikelstrahlanlage in Verbindung steht, und der es ermöglicht, dass Strahlparameter, welche zur Steuerung des Partikelstrahls verwendet werden, in der Datenaufnahmeeinheit 45 zu speichern. Die Steuerungseinheit 59 steuert beispielsweise die Scan-Magnete 61 an, welche zur Steuerung des Bestrahlungsverlaufs eingesetzt werden.

Weiterhin verfügt die Datenaufnahmeeinheit 45 über einen vierten Eingang 63, über welchen ein Bewegungssignal, das von einem Bewegungssensor 65 erfasst worden ist und das einer Bewegungserfassungseinheit 67 der Partikelstrahlanlage weitergeleitet wird, gespeichert werden kann.

Weitere Untereinheiten der Datenaufnahmeeinheit 45 wie ein nicht explizit dargestellter zentraler Prozessor und nicht explizit dargestellte Speichereinheiten sind standardmäßig vorgesehen.

Die Speicherung der unterschiedlichen Signale und Messdaten, welche über die verschiedenen Eingänge der Datenaufnahmeeinheit 45 zur Verfügung gestellt werden, erfolgt insbesondere zeitlich miteinander korreliert, so dass bei einer anschließenden Auswertung festgestellt werden kann, welcher Bewegungszustand, welche Phase des Bestrahlungsverlaufs und/oder welche Strahleigenschaft bei der Aufzeichnung der Messdaten vorgelegen hat. Das bedeutet, das beispielsweise jedes Signal und jegliche Messdaten eine Zeitinformation aufweisen, mit das jeweilige Signal oder der jeweilige Messwert einer bestimmten Zeit zugeordnet werden kann.

Das gesamte Bewegungserfassungsystem ist hier beispielhaft lediglich mit einem externen Bewegungssensor 65 dargestellt. Verschiedene Bewegungserfassungssysteme können eingesetzt werden: Z.B. kann die Bewegung der Bauchdecke mit Hilfe der Messung einer Bewegungsamplitude eines Infrarotmarkers mit einem Kamerasystem detektiert werden. Es ist auch möglich, die Bewegungsphase aus der Dehnung eines um den Bauch oder den Brustkorb geschnallten Sensors zu ermitteln. Wird das Verfahren der Spirometrie verwendet, atmet der Patient durch einen Volumensensor, mit dem zeitlich aufgelöst das Volumen der ein- und ausgeatmeten Luft bestimmt werden kann. Auch ein Temperatursensor, der über den Verlauf der Atmung Auskunft gibt, kann vorgesehen sein. Die Bewegungserfassung kann auch mit Hilfe eines kleinen, reiskorngroßen elektromagnetischen Transponders, welcher in dem Zielvolumen implantiert ist, erfolgen.

Auch Bildgebungssysteme, wie z.B. Ultraschall oder Fluoroskopie, können zur Überwachung der Bewegung des Zielvolumens eingesetzt werden, wobei beispielsweise implantierte Marker verwendet werden und die Erfassung der Bewegung unterstützen, indem diese mit dem Bildgebungssystem detektiert werden können.

Weiterhin kann vorgesehen sein, dass als externer Bewegungssensor 65 ein radioaktiver Marker, der z.B. auf der Körperoberfläche angebracht ist, über das PET-System 25 selbst detektiert wird.

Die Auswertung der Messdaten erfolgt in einer Auswertungseinheit 69. Die Auswertungseinheit 69 ist dabei in spezieller Weise so ausgebildet, dass mit ihr - im Gegensatz zu herkömmlichen bei Partikelstrahlanlagen eingesetzten Systemen - die aufgezeichneten Messdaten insbesondere zeitaufgelöst und bewegungskorrigiert ausgewertet werden können. Auf diese Weise lässt sich eine bewegungskorrigierte Aktivitätsverteilung ermitteln, welche nun auch bei einem bewegten Zielvolumen Rückschlüsse auf die tatsächlich deponierte Dosisverteilung erlaubt.

Weiterhin steht das PET-System 25 mit einer Kontrollkonsole 71 in Verbindung bzw. ist mit dieser gekoppelt, welche es einem Anwender erlaubt, eine Bestrahlung zu initiieren und den Bestrahlungsverlauf zu steuern und zu überwachen.

Weiterhin steht das PET-System 25 mit einem Bestrahlungsplanungssystem 73 in Verbindung bzw. ist mit dieser gekoppelt, so dass die aufgezeichnete Aktivitätsverteilung mit den in der Bestrahlungsplanung enthaltenen Parametern oder mit den aus der Bestrahlungsplanung ermittelbaren Größen verglichen werden kann. Ein derartiger Vergleich kann beispielsweise in einer weiteren, eigens dafür ausgebildeten Beurteilungseinheit 75, welche die Daten des Bestrahlungsplanungssystems 73 mit den Daten des PET-Systems 25 in Beziehung setzt, durchgeführt werden.

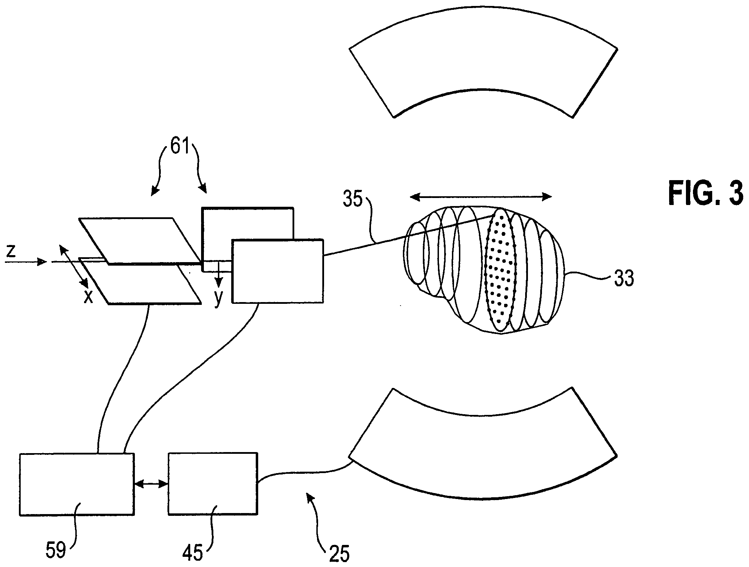

Fig. 3 zeigt schematisch die Anordnung des PET-Systems um ein Zielvolumen, das mit einem Scan-Verfahren bestrahlt wird. Die Bestrahlung mit einem Scan-Verfahren umfasst dabei einen Partikelstrahl 35, welcher so dimensioniert ist, dass in dem Zielvolumen 33 lediglich an einem kleinen, umschriebenen Bezirk eine Einzeldosis deponiert werden kann. Ein solcher kleiner Bezirk ist ein Rasterpunkt, wobei die Parameter der Rasterpunkte vorzugsweise in der Bestrahlungsplanung enthalten sind. Um das gesamte Zielvolumen 33 zu bestrahlen, werden sukzessive unterschiedliche Orte des Zielvolumens 33, insbesondere die unterschiedliche Rasterpunkte, nacheinander bestrahlt. Der Partikelstrahl 35 wird dabei mithilfe von Scan-Magneten 61 abgelenkt und so über das Zielvolumen gescannt. Die Rasterpunkte können hierbei abgescannt oder abgetastet werden. Zur Bestrahlung unterschiedlicher Iso-Energieschichten wird die Energie des Partikelstrahls 35 passend eingestellt. Gezeigt ist ein Zielvolumen 33, bei dem drei distale Iso-Energieschichten a, b, c bereits bestrahlt worden sind, und bei denen der Partikelstrahl 35 über die nachfolgende Iso-Energieschicht d scannt.

Es sind verschiedene Scan-Verfahren bekannt, wie z.B. das Raster-Scanning, bei dem der Strahl ohne Abschaltung zwischen benachbarten Rasterpunkten über das Zielvolumen scannt, das Spot-Scanning, bei dem eine Abschaltung zwischen Zielpunkten erfolgt, oder kontinuierliche Scann-Verfahren, bei denen der Strahl kontinuierlich abgelenkt wird.

Gegebenenfalls kann noch eine der Übersichtlichkeit halber nicht gezeigte passive oder aktive Energiemodulationsvorrichtung vorgesehen sein. Diese kann beispielsweise zwischen den Scan-Magneten 61 und dem Zielvolumen 33 angeordnet sein, wobei mit der Energiemodulationsvorrichtung die Eindringtiefe des Partikelstrahls 35 einer Bewegung des Zielvolumens 33 nachgeregelt werden kann. Alternativ und/oder zusätzlich kann eine Energiemodulationsvorrichtung verwendet werden, die in Strahlrichtung gesehen vor den Scan-Magneten angeordnet ist. Eine derartige Energiemodulationsvorrichtung kann beispielsweise zur Einstellung der Energie des Partikelstrahls für verschiedene Iso-Energieschichten verwendet werden. Letzteres Verfahren wird insbesondere bei Partikelstrahlanlagen mit einem Zyklotron angewendet.

Durch den Scan-Prozess entsteht folglich eine zeitlich protrahierte Bestrahlung, während der die zu deponierende Dosis inkrementell deponiert wird. Diese Dosis erzeugt eine inkrementell anwachsende Aktivitätsverteilung, die zeitaufgelöst durch das PET-System 25 erfasst werden kann.

Dadurch, dass das PET-System 25 die Messdaten zeitaufgelöst aufzeichnet und mit der Steuerungseinheit 59, welche den Scan-Verlauf steuert, in Wirkverbindung steht, kann die ermittelte Aktivitätsverteilung stets zu dem aktuellen Scan-Verlauf in Beziehung gesetzt werden. Dies ist selbst dann möglich, wenn sich das Zielvolumen 33 bewegt. Der Bestrahlungsverlauf kann so mit Hilfe des PET-Systems 25 überwacht und gegebenenfalls gesteuert werden, selbst bei komplexen Bestrahlungsvorgängen wie bei dem Scan-Verfahren bei einem bewegten Zielvolumen.

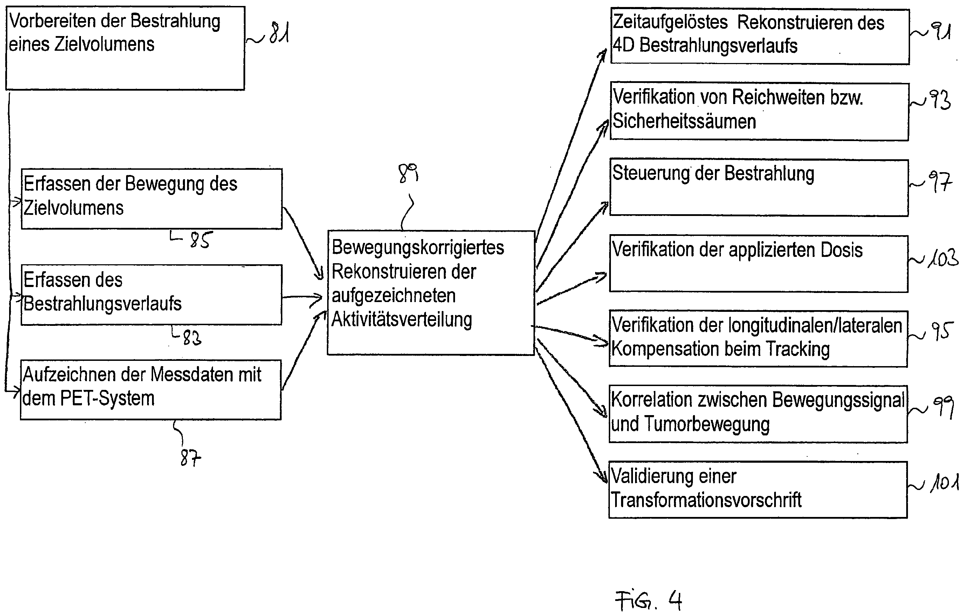

Fig. 4 zeigt einen schematischen Überblick über verschiedene Verfahrensschritte, welche in einer Ausführungsform des Verfahrens durchgeführt werden.

Zu Beginn wird die Bestrahlung des Zielvolumens vorbereitet (Schritt 81). Anschließend wird die Bestrahlung gestartet. Dabei wird der Bestrahlungsverlauf erfasst (Schritt 83). Weiterhin wird während der Bestrahlung die Bewegung des Zielvolumens 33 erfasst (Schritt 85). Zudem werden die durch die Bestrahlung im Zielvolumen 33 erzeugten Photonen mit Hilfe des PET-Systems 25 zeitaufgelöst aufgezeichnet (Schritt 87) .

Während bzw. nach erfolgter Bestrahlung werden die aufgezeichneten PET-Daten ausgewertet und die den Daten zu Grunde liegende Aktivitätsverteilung im Zielobjekt 31 ermittelt. Die Rekonstruktion der Aktivitätsverteilung erfolgt dabei bewegungskorrigiert (Schritt 89). Dies bedeutet, dass bei der Rekonstruktion die Bewegung des Zielobjekts berücksichtigt wird, so dass eine Unschärfe bei der Ermittlung der räumlichen Charakteristik der Aktivitätsverteilung, die auf eine Bewegung des Zielobjekts 31 während der Aufzeichnung der Messdaten zurückzuführen ist, reduziert wenn nicht gar eliminiert wird.

Im Folgenden werden einige Anwendungen beschrieben, die im Anschluss daran einzeln und/oder in Kombination durchgeführt werden können.

### Monitoring/Rekonstruktion des 4D-Bestrahlungsablaufs (Schritt 91)

Dies wurde bereits vorstehend beschrieben. Insbesondere lassen sich das PET-Signal bzw. die gemessene Aktivitätsverteilung mit zumindest einem Teil der Rasterpunkte, der Iso-Energieschichten a, b, c, d und/oder der aus einem Beschleuniger wie aus einem Synchrotron 15 extrahierten Strahlpulse (auch als Spill bezeichnet) korrelieren. Fehlerhaft deponierte Einzeldosen können z.B. auf diese Weise identifiziert werden.

### Verifikation von Sicherheitssäumen (Schritt 93)

Das Verfahren kann beispielsweise zur Verifikation von Sicherheitssäumen eingesetzt werden. Sicherheitssäume werden bei der Bestrahlung von Zielvolumina 33, insbesondere von bewegten Zielvolumina 33, eingesetzt, um unter anderem zu sichern, dass bewegte Zielvolumina 33 vollständig mit einer verordneten Dosis belegt werden, welche zuvor in der Bestrahlungsplanung ermittelt und abgelegt wurde. Da die räumliche Charakteristik der deponierten Dosisverteilung mit Hilfe des Positronen-Emissions-Tomographen 25 bewegungskorrigiert ermittelt werden kann, zeigt die rekonstruierte Aktivitätsverteilung in 4D die Abdeckung des Zielgebiets.

Ein geeignetes und therapeutisch äußerst bedeutsames Beispiel ist dabei die Bestrahlung von Lungentumoren. Durch den erheblichen Dichteunterschied zwischen Tumorgewebe und gesundem Lungengewebe lassen sich so genannte Überreichweiten feststellen.

Überreichweiten treten dabei an denjenigen Stellen auf, die im Sicherheitssaum liegen, an denen jedoch der Tumor während der Bestrahlung nur teilweise zu liegen kommt. Überreichweiten treten daher vor allem an den lateralen Tumorrändern - bezogen auf die Einfallsrichtung des Partikelstrahls - auf, und zeichnen sich dadurch aus, dass in diesen Regionen der Partikelstrahl 35 aufgrund der geringeren Dichte des Gewebes tiefer in das Zielobjekt 31 eindringt.

Mithilfe des PET-Systems 25 können diese Überreichweiten selbst bei einem bewegten Zielobjekt detektiert werden, ohne dass eine Bewegung des Zielobjekts 31 die Auswertung der Messdaten stört. Wenn Überreichweiten vollständig verschwinden, kann davon ausgegangen werden, dass die Sicherheitssäume bestenfalls exakt ausreichend oder aber - wahrscheinlicher - sogar zu klein waren. In diesem Fall sollte die Messung am Tumor genauer betrachtet werden, um festzustellen, ob in dem Tumor tatsächlich die gewünschte Dosisverteilung deponiert wurde.

### Verifikation der Reichweite (Schritt 93)

Das Verfahren kann auch zur Verifikation der Reichweite eingesetzt werden. Insbesondere bei der Bestrahlung von bewegten Zielobjekten 31 werden Verfahren wie Tracking oder Gating verwendet. Bei diesen Verfahren sind keine - oder nur kleine - distale Überschreitungen des Zielvolumens 33 zu erwarten, die üblicherweise durch die Bestrahlungsplanung festgelegt werden und hieraus bekannt sind. Beim Rescanning wie auch bei passiver Strahlapplikation (Strahlapplikation mit einem dem Zielvolumen angepassten Kollimator und/oder Energiemodulator) mit Sicherheitssäumen werden Reichweiten erwartet, die über die distale Kante des Zielvolumens 33 hinausgehen.

Prinzipiell kann die Reichweite bei diesen bewegten Zielobjekten 31 und Zielvolumina 33 nun überprüft werden. Wenn die zeitliche Entstehung der Aktivitätsverteilung zusätzlich erfasst und mit dem Bestrahlungsverlauf korreliert wird, ist sogar eine Aussage möglich, was im Falle von Abweichungen die Ursache war, d.h. bei welchem Rasterpunkt, bei welcher Iso-Energieschicht, usw. die Abweichung aufgetreten ist.

Die Analyse, wann wo welche Zieldosis ggf. nicht korrekt deponiert wurde, kann Aufschluss darüber geben, wie die Bestrahlungseinrichtung 10 gegebenenfalls optimiert werden kann.

### Verifikation der longitudinalen/lateralen Kompensation beim Tracking (Schritt 95)

Beim Tracking insbesondere in longitudinaler Richtung - d.h. in Strahlrichtung - wird z.B. während der Bestrahlung die Reichweite des Strahls 35 angepasst. Dies kann je nach Ausführung des Tracking-Systems über mechanische Komponenten erfolgen, beispielsweise über Absorberkeile, die die Energie des Partikelstrahls 35, kurz als Strahlenergie bezeichnet, variabel modulieren. Hierbei kann es zu einer verzögerten Reichweitenmodulation kommen, da die mechanischen Komponenten die Strahlenergie unter Umständen nicht ausreichend schnell oder nicht ausreichend beschleunigt modulieren können. Dies kann beispielsweise auftreten, wenn die Strahlapplikation aufgrund hoher Dosisleistung sehr schnell erfolgt und/oder wenn die von Rasterpunkt zu Rasterpunkt erforderliche Modulation der Energie des Partikelstrahls 35 zu große Amplitudenveränderungen aufweist.

Durch die zeitliche Korrelation der entstehenden Aktivitätsverteilung mit dem Bestrahlungsverlauf kann nun festgestellt werden, welche Kombination aus Rasterpunkt und Bewegungsphase beim Tracking zu Problemen führt. Durch die zeitliche Korrelation zu allen anderen Teilsystemen kann außerdem die zeitliche Verzögerung gemessen werden und für entsprechende Vorhersagenmodelle verwendet werden. Diese Vorhersagemodelle können wiederum in der Bestrahlungsplanung verwendet werden. Hierdurch kann eine Bestrahlung insgesamt optimiert werden.

Entsprechendes gilt prinzipiell auch für ein laterales Tracking, d.h. eine laterale Anpassung der Strahllage an die Bewegung des Zielobjekts 31, wobei das laterale Tracking in der Regel einfacher und schneller durchzuführen ist, da für das laterale Tracking keine mechanisch angesteuerten Komponenten verwendet werden.

### Steuerung der Bestrahlung/adaptive Therapie (Schritt 97)

Die mit dem PET-System 25 gemessene deponierte Aktivitätsverteilung kann zu unmittelbaren bzw. zur mittelbaren Steuerung der Bestrahlung (bezeichnet als Bild geführte Strahlentherapie) verwendet werden.

Wenn die entstehende Dosisverteilung während der Bestrahlung mit dem PET-System 25 gemessen wird, kann die Bestrahlung z.B. abgebrochen werden, sobald eine zu große Abweichung von einem Sollwert gemessen wird.

Wenn bei einer fraktionierten Bestrahlung, das heißt einer Bestrahlung des Zielvolumens 33 in mehreren in sich angeschlossenen Bestrahlungssitzungen, nach Durchführung einer Fraktion die tatsächlich deponierte Dosisverteilung dieser Fraktion mit dem PET-System 25 gemessen wird, kann für nachfolgende Fraktionen aufgrund dieser Kenntnis die Bestrahlung angepasst werden, so dass eine insgesamt zu deponierende Dosisverteilung erreicht wird.

Bei der Bestrahlung eines bewegten Zielvolumens 33 mit Rescanning und insbesondere bei der Bestrahlung mit Sicherheitssäumen liegt die Annahme zu Grunde, dass die Wechselwirkung zwischen Strahl- und Organbewegung zwar zu einer inhomogenen Dosisverteilung führen kann, dass sich die inhomogene Dosisverteilung im statistischen Mittel über eine Fraktion oder über mehrere Fraktionen ausmittelt. Mithilfe der erfindungsgemäßen Vorrichtung oder mithilfe des erfindungsgemäßen Verfahrens ist es nun möglich, Rückschlüsse auf die tatsächlich applizierte Dosisverteilung zu ziehen, um sicherzustellen, dass die gewünschte Mittelungseffekte tatsächlich aufgetreten sind.

Durch die zeitliche Korrelation der entstehenden Aktivitätsverteilung mit dem Bestrahlungsverlauf kann insbesondere analysiert werden, welche Bestrahlungsphase zu welchen Effekten führt. Parallel dazu kann die 4D-Dosisverteilung berechnet werden, die auf Basis der Bewegungstrajektorie und des Scan-Fortschritts bestimmt wird, also ein Vorhersagewert für die Dosisverteilung, die mit dem PET-System 25 gemessen werden kann. Dies erlaubt einen direkten, abschnittsweisen Vergleich bei den sukzessive ablaufenden Bestrahlungsphasen zwischen der tatsächlich deponierten Dosisverteilung und der zu erwartenden Dosisverteilung.

Es ist möglich und sinnvoll, die deponierte Aktivitätsverteilung nicht nur kumulativ zu messen, indem z.B. die deponierte 4D-Aktivitätsverteilung für einen Referenzzustand kumulativ ermittelt wird, sondern die deponierte Aktivitätsverteilung pro Bewegungsphase zu messen, d.h. also als multiple 3D-Aktivitätsverteilungen. Beispielsweise sollte dann bei einer Bestrahlung im Gating-Verfahren der ansteigende Aktivierungsgrad nur während derjenigen Bewegungsphasen zunehmen, die innerhalb des Gating-Fensters liegen.

### Korrelation zwischen der Bewegung des Zielvolumens 33 und der Bewegungserfassungseinrichtung 65 (Schritt 99)

Bei bestimmten Bestrahlungsverfahren wie dem Gating und dem Tracking wird die Bestrahlung oftmals mithilfe eines Bewegungssignals gesteuert. Das Bewegungssignal charakterisiert die Bewegung des Zielvolumens 33 insofern, als das aus dem Signal auf den Bewegungszustand des Zielvolumens 33 geschlossen werden kann. Diesem Schluss liegt die Annahme zu Grunde, dass das Bewegungssignal und die Bewegung des Zielvolumens 33 zueinander korreliert sind. Falls diese Korrelation aus bestimmten Gründen nicht mehr gegeben ist, würde dies im Allgemeinen dazu führen, dass die Dosis fehlerhaft im Zielvolumen 33 deponiert wird.

Der Fall, dass sich die Korrelation zwischen Bewegungssignal und Bewegung des Zielvolumens 33 ändert, tritt in der Praxis jedoch mitunter auf. Beispielsweise kann sich die Güte der Korrelation innerhalb von kurzen Zeiträumen ändern - z.B. durch Husten eines Patienten mit einem Bronchialkarzinom. Auch kann sich die Anatomie z.B. durch eine sich entspannende Muskulatur graduell ändern, so dass die Korrelation der Bewegung und des Bewegungssignals einen Drift erfährt.

Die durch das PET-System 25 zeitaufgelöst aufgezeichneten Messdaten erlauben jedoch eine Detektion einer derartigen Abweichung. Insbesondere, wenn die Messdaten während der Bestrahlung aufgezeichnet werden und mit dem Bestrahlungsverlauf korreliert werden, verhindert dies, dass dem Bestrahlungsverlauf und dem Aufzeichnen der Messdaten durch das PET-System 25 eine unterschiedliche Korrelation zu Grunde liegt.

Doch auch im letzteren Fall kann es - wenn eine fehlerhafte Korrelation zwischen Bewegungssignal und Bewegung vorliegt - zu einer fehlerhaften Auswertung der deponierten Dosisverteilung kommen, da ein unzureichendes Bewegungssignal verwendet wird. Aufgrund des Aufbaus des Zielvolumens, z.B. aufgrund der besonderen Anatomie der Lunge mit dichtem Tumorgewebe und lockeren Lungengewebe, kann man jedoch davon ausgehen, dass über die vierdimensional rekonstruierte Aktivitätsverteilung eine interne Bewegungstrajektorie rekonstruiert werden kann, da der Teilchenstrahl in dichtem Tumorgewebe mehr β⁺-Emitter erzeugt als zum Beispiel im dünnen Lungengewebe. Auf diese Weise kann aus den Messdaten das Zielvolumen 33 zeitlich verfolgt werden. Eine Verifikation der Korrelation zwischen Bewegungssignal und Bewegung kann nun erfolgen.

In speziellen Ausführungen ist es möglich und sinnvoll, beispielsweise einen radioaktiven Marker auf der Hautoberfläche anzubringen, dessen Aktivität in den Messdaten sichtbar ist und der somit zur zeitlichen Bewegungsbestimmung verwendet werden kann. Hierdurch ist es möglich, mit dem PET-System zusätzlich eine Bewegungsgröße zu messen.

### Validierung einer Transformationsvorschrift (Schritt 101)

Bei 4D-Bestrahlungen und 4D-Bestrahlungsplanungen liegt dem Bestrahlungsplan bzw. der Bestrahlung oftmals eine Transformationsvorschrift zu Grunde, welche den Übergang zwischen den verschiedenen Bewegungsphasen beschreibt und die Anpassung der Bestrahlung an die verschiedenen Bewegungsphasen erlaubt. Im Verlauf der Bestrahlung, insbesondere bei einer fraktionierten Bestrahlung, kann sich diese Transformationsvorschrift jedoch ändern, da die anatomische und physiologische Situation sich im Patienten ändern kann. Durch die erfindungsgemäße Vorrichtung kann die Transformationsvorschrift kalibriert werden, indem die Geometrie der gemessenen Aktivitätsverteilung bzw. Dosisverteilung z.B. in einer Referenzphase verglichen wird. Weiterhin kann beurteilt werden, ob sich die Anatomie in der Referenzphase geändert hat.

### Verifikation der applizierten Dosis (Schritt 103)

Aus der gemessenen Aktivitätsverteilung kann Rückschluss darüber erhalten werden, wie die Dosis tatsächlich im Zielvolumen 33 appliziert wurde. Insbesondere kann aus der Aktivitätsverteilung eine räumliche Charakteristik der tatsächlich applizierten Dosis ermittelt werden.

## Patentansprüche

1. Bestrahlungsanlage mit einer Vorrichtung zur Auswertung einer in einem bewegten Zielobjekt (31) auftretenden Aktivitätsverteilung, wobei die Bestrahlungsanlage eine Bestrahlungseinrichtung (10), mit der ein Strahl (35) erzeugbar ist, umfasst, und wobei die Aktivitätsverteilung durch den mit der Bestrahlungseinrichtung (10) erzeugten Strahl (35) erzeugbar ist, wobei die Bestrahlungseinrichtung eine zweite Steuerungseinheit (59) aufweist und wobei mit der zweiten Steuerungseinheit ein Verlauf einer Bestrahlung durch den Strahl steuerbar ist, die Vorrichtung zur Auswertung aufweisend:
- einen Positronen-Emissions-Tomographen (25), welcher ausgebildet ist zum Aufzeichnen von durch den Strahl im Zielobjekt erzeugten Photonen und zur Erzeugung von Messdaten, welche Entstehungsorte der Photonen repräsentieren, mit einer ersten Steuerungseinheit,
- eine Bewegungserfassungseinrichtung (65), welche ausgebildet ist, ein Bewegungssignal zu erzeugen, das die Bewegung des Zielobjekts repräsentiert,
- eine Datenaufnahmeeinheit (45) zur Steuerung der Datenaufnahme des Positronen-Emissions-Tomographen mit
∘ einem ersten Eingang (49) zur Erfassung der Messdaten des Positronen-Emissions-Tomographen,
∘ einem zweiten Eingang (53) zum Eingeben von die Strahleigenschaften betreffenden Daten in die Datenaufnahmeeinheit,
∘ einem dritten Eingang (57), der mit der zweiten Steuerungseinheit (59) der Bestrahlungseinrichtung verbunden ist, zum Eingeben von Strahlparametern, welche zur Steuerung des Partikelstrahls verwendet werden, in die Datenaufnahmeeinheit,
∘ einem vierten Eingang (63) zur Erfassung des mit der Bewegungserfassungseinrichtung erzeugten Bewegungssignals,
wobei die Datenaufnahmeeinheit ferner hergerichtet ist zur zeitlich korrelierten Speicherung der an den Eingängen erfassten Signale und Messdaten,
- eine Auswertungseinheit (69), welche ausgebildet ist, die Entstehungsorte der gemessenen Photonen zu Positionen im Zielobjekt unter Verwendung des Bewegungssignals zuzuordnen, wodurch eine räumliche Charakteristik der tatsächlich im Zielobjekt erzeugten Aktivitätsverteilung über die durch den Strahl erzeugten Photonen zeitaufgelöst und bewegungskorrigiert auswertbar ist,
- wobei die Steuerung des Bestrahlungsverlaufs durch die zweite Steuerungseinheit gleichzeitig die Steuerung der Datenaufzeichnung mit dem Positronen-Emissions-Tomographen durch die Datenaufnahmeeinheit bewirkt, so dass die mit der Auswertungseinheit ermittelte zeitaufgelöste und bewegungskorrigierte Aktivitätsverteilung zu dem Bestrahlungsverlauf in Beziehung gesetzt werden kann.

2. Vorrichtung nach Anspruch 1,
wobei die Auswertungseinheit (69) zusätzlich ausgebildet ist, die Messdaten zeitlich mit sukzessive ablaufenden Bestrahlungsphasen zu korrelieren, wodurch eine sich ändernde räumliche Charakteristik der erzeugten Aktivitätsverteilung im Zielobjekt zu den sukzessive ablaufenden Bestrahlungsphasen in Beziehung gesetzt wird.

3. Vorrichtung nach einem der Ansprüche 1 oder 2, wobei die Auswertungseinheit (69) ausgebildet ist, um das Bewegungssignal oder ein zusätzliches Bewegungssignal, welches ebenso die Bewegung des Zielobjekts (31) kennzeichnet, aus den aufgezeichneten Messdaten zu ermitteln, insbesondere mit Hilfe eines radioaktiven Markers, der durch die Bewegung des Zielobjekt bewegt wird.

4. Vorrichtung nach einem der Ansprüche 1 bis 3,
wobei die Auswertungseinheit (69) zusätzlich derart ausgebildet ist, aus einer Intensität der gemessenen Photonen quantitativ ein Maß für eine im Zielobjekt (31) deponierte Dosisverteilung an den Positionen im Zielobjekt zu ermitteln.

5. Vorrichtung nach einem der Ansprüche 1 bis 4,
wobei die Vorrichtung zusätzlich aufweist:
- eine Beurteilungseinheit (75), welche ausgebildet ist, die ausgewertete Aktivitätsverteilung mit einer charakteristischen Größe einer Bestrahlungsplanung zu vergleichen.

6. Verfahren zur Auswertung einer in einem nicht belebten Zielobjekt (31) erzeugten Aktivitätsverteilung, wobei unter Verwendung einer Vorrichtung nach einem der Ansprüche 1 bis 5 eine räumliche Charakteristik der im Zielobjekt durch einen Strahl (35) erzeugten Aktivitätsverteilung mit Hilfe einer räumlichen Verteilung der durch den Strahl induzierten Photonen, welche zeitlich aufgelöst gemessenen werden, bestimmt wird.

7. Verfahren nach Anspruch 6,
wobei zusätzlich aus einer Intensität der gemessenen Photonen quantitativ ein Maß für eine deponierte Dosisverteilung an den Positionen im Zielobjekt (31) ermittelt wird.

8. Verfahren nach Anspruch 6 oder 7,
wobei aus der Verteilung der zeitlich aufgelöst gemessenen Photonen eine Bewegung des Zielobjekts (31) ermittelt wird.

9. Verfahren nach einem der Ansprüche 6 bis 8, wobei die zeitlich aufgelöst gemessenen Photonen zu verschiedenen Bewegungsphasen zugeordnet werden und wobei eine räumliche Charakteristik im Zielobjekt deponierten Dosisverteilung für die verschiedenen Bewegungsphasen separat ausgewertet wird.

10. Verfahren nach einem der Ansprüche 6 bis 9, wobei aus einem Bestrahlungsplan eine zu erwartende Verteilung von Photonen bestimmt wird, welche mit der gemessenen räumlichen Verteilung von Photonen verglichen wird.

## Claims

1. An irradiation apparatus comprising a device for evaluating an activity distribution occurring in a moved target object (31), wherein the irradiation apparatus comprises an irradiation device (10) adapted to generate a beam (35), and wherein said activity distribution can be produced by the beam (35) generated by said irradiation device (10); wherein the irradiation device includes a second control unit (59) and wherein said second control unit is adapted to control a course of irradiation by the beam;
wherein said device for evaluating comprises:
a positron emission tomography device (25) adapted to record photons generated in the target object by said beam and to generate measurement data representing points of origin of the photons, comprising a first control unit;
- a movement capturing device (65) adapted to generate a movement signal which represents the movement of the target object;
a data acquisition unit (45) for controlling the data acquisition of the positron emission tomography device, comprising
∘ a first input (49) for acquiring the measurement data of the positron emission tomography device;
∘ a second input (53) for inputting, into the data acquisition unit, data related to the beam characteristics;
∘ a third input (57) which is connected to the second control unit (59) of the irradiation device, for inputting, into the data acquisition unit, beam parameters used to control the particle beam;
∘ a fourth input (63) for acquiring the movement signal generated by the movement capturing device;
wherein said data acquisition unit is further adapted to store the signals and measurement data acquired at the inputs in time-correlated manner;
- an evaluation unit (69) adapted to associate the points of origin of the measured photons with positions in the target object using the movement signal, whereby, through the photons generated by the beam, a spatial characteristic of the activity distribution actually generated in the target object can be evaluated in time-resolved and movement-corrected manner;
- wherein the controlling of the course of irradiation by the second control unit simultaneously causes the data acquisition unit to control the data acquisition by the positron emission tomography device, so that the time-resolved and movement-corrected activity distribution determined by the evaluation unit can be related to the course of irradiation.

2. The device according to claim 1,
wherein the evaluation unit (69) is additionally adapted to correlate, in terms of time, the measurement data with successively proceeding irradiation phases, thereby relating a varying spatial characteristic of the generated activity distribution in the target object to the successively proceeding irradiation phases.

3. The device according to any one of claims 1 or 2, wherein the evaluation unit (69) is adapted to determine the movement signal or an additional movement signal which is also indicative of the movement of the target object (31) from the acquired measurement data, in particular by means of a radioactive marker which is moved due to the movement of the target object.

4. The device according to any one of claims 1 to 3, wherein the evaluation unit (69) is additionally adapted to quantitatively determine, from an intensity of the measured photons, a measure for a dose distribution deposited in the target object (31) at the positions in the target object.

5. The device according to any one of claims 1 to 4, wherein the device additionally comprises:
- an assessment unit (75) which is adapted to compare the evaluated activity distribution with a characteristic parameter of a treatment plan.

6. A method for evaluating an activity distribution generated in a non-living target object (31), wherein by using a device according to any one of claims 1 to 5, a spatial characteristic of the activity distribution generated in the target object by a beam (35) is determined using a spatial distribution of the photons induced by the beam, which is measured in time-resolved manner.

7. The method according to claim 6,
wherein, additionally, a measure for a dose distribution deposited at the positions in the target object (31) is quantitatively determined from an intensity of the measured photons.

8. The method according to claim 6 or 7,
wherein a movement of the target object (31) is determined from the distribution of the photons measured in time-resolved manner.

9. The method according to any one of claims 6 to 8, wherein the photons measured in time-resolved manner are associated with different movement phases, and wherein a spatial characteristic of the dose distribution deposited in the target object is evaluated separately for the different movement phases.

10. The method according to any one of claims 6 to 9, wherein a distribution of photons to be expected is determined from an irradiation plan, which is compared to the measured spatial distribution of photons.

## Revendications

1. Installation d'irradiation comprenant un dispositif d'évaluation d'une répartition d'activité apparaissant dans un objet cible (31) en mouvement, sachant que l'installation d'irradiation comporte un dispositif d'irradiation (10) permettant de générer un faisceau (35), et sachant que la répartition d'activité peut être produite avec le faisceau (35) généré par le dispositif d'irradiation (10), le dispositif d'irradiation présentant une deuxième unité de commande (59) et cette deuxième unité de commande permettant de contrôler l'évolution d'une irradiation par le faisceau, le dispositif d'évaluation présentant :
- un tomographe à émission de positons (25) qui est conçu pour enregistrer des photons produits par le faisceau dans l'objet cible et pour générer des données de mesure qui représentent les lieux de formation des photons, comprenant une première unité de commande,
- un dispositif de détection de mouvement (65) qui est conçu pour générer un signal de mouvement qui représente le mouvement de l'objet cible,
- une unité d'acquisition de données (45) destinée à commander l'acquisition de données du tomographe à émission de positons, comprenant :
∘ une première entrée (49) destinée à recueillir les données de mesure du tomographe à émission de positons,
∘ une deuxième entrée (53) destinée à saisir des données concernant les propriétés du faisceau, dans l'unité d'acquisition de données,
∘ une troisième entrée (57) qui est reliée à la deuxième unité de commande (59) de l'installation d'irradiation, à des fins d'introduction, dans l'unité d'acquisition de données, de paramètres de faisceau utilisés pour la commande du faisceau de particules,
∘ une quatrième entrée (63) destinée à recueillir le signal de mouvement produit par le dispositif de détection de mouvement, sachant que l'unité d'acquisition de données est en outre préparée pour une sauvegarde en corrélation temporelle des signaux et données de mesure recueillis aux entrées,
- une unité d'évaluation (69) qui est conçue pour associer les lieux de formation des photons mesurés à des positions dans l'objet cible, en utilisant le signal de mouvement, ce qui permet d'évaluer une caractéristique spatiale de la répartition d'activité effectivement produite dans l'objet cible, par le biais des photons produits par le faisceau, avec une résolution temporelle et une correction du mouvement,
- sachant que la commande de l'évolution de l'irradiation par la deuxième unité de commande a en même temps pour effet le contrôle de l'enregistrement des données avec le tomographe à émission de positons, par l'unité d'acquisition de données, de sorte que la répartition d'activité déterminée par l'unité d'évaluation, avec résolution temporelle et correction du mouvement, peut être mise en relation avec l'évolution de l'irradiation.

2. Dispositif selon la revendication 1, dans lequel l'unité d'évaluation (69) est en outre conçue pour mettre les données de mesure en corrélation temporelle avec des phases d'irradiation se déroulant successivement, ce qui a pour effet de mettre une caractéristique spatiale variable de la répartition d'activité produite dans l'objet cible en relation avec les phases d'irradiation successives.

3. Dispositif selon l'une des revendications 1 ou 2, dans lequel l'unité d'évaluation (69) est conçue pour déterminer le signal de mouvement ou un signal de mouvement supplémentaire qui représente également le mouvement de l'objet cible (31), à partir des données de mesure enregistrées, notamment à l'aide d'un traceur radioactif qui est déplacé par le mouvement de l'objet cible.

4. Dispositif selon l'une des revendications 1 à 3, dans lequel l'unité d'évaluation (69) est en outre conçue pour déterminer, à partir d'une intensité des photons mesurés, de manière quantitative une valeur d'une distribution de dose déposée dans l'objet cible (31), aux positions dans l'objet cible.

5. Dispositif selon l'une des revendications 1 à 4, dans lequel le dispositif présente en outre :
- une unité d'appréciation (75) qui est conçue pour comparer la répartition d'activité évaluée avec une grandeur caractéristique d'un plan d'irradiation.

6. Procédé d'évaluation d'une répartition d'activité produite dans un objet cible (31) inanimé, selon lequel on utilise une installation conforme à l'une des revendications 1 à 5 pour déterminer une caractéristique spatiale de la répartition d'activité produite par un faisceau (35) dans l'objet cible, à l'aide d'une distribution spatiale des photons induits par le faisceau qui sont mesurés avec une résolution temporelle.

7. Procédé selon la revendication 6, selon lequel on détermine en outre, à partir d'une intensité des photons mesurés, de manière quantitative une valeur d'une distribution de dose déposée, aux positions dans l'objet cible (31).

8. Procédé selon la revendication 6 ou 7, selon lequel on détermine un mouvement de l'objet cible (31) à partir de la répartition des photons mesurés avec une résolution temporelle.

9. Procédé selon l'une des revendications 6 à 8, selon lequel les photons mesurés avec une résolution temporelle sont associés à des phases de mouvement différentes, et selon lequel une caractéristique spatiale de la distribution de dose déposée dans l'objet cible est évaluée séparément pour les différentes phases de mouvement.

10. Procédé selon l'une des revendications 6 à 9, selon lequel on détermine, à partir d'un plan d'irradiation, une répartition attendue des photons, qui est comparée avec la distribution spatiale mesurée des photons.
